# EUROPEAN PATENT SPECIFICATION

(11) **EP 1 698 364 B1**
(45) Date of publication and mention of the grant of the patent: **30.07.2008**
(21) Application number: 04766947.8
(22) Date of filing: 20.09.2004
(51) Int. Cl.: A61M 5/315

(54) **UNIT FOR THE MANUAL OR AUTOMATIC INJECTION OF INJECTABLE PRODUCTS**
GERÄT FÜR DIE MANUELLE ODER AUTOMATISCHE INJEKTION VON INJEKTIONSPRÄPARATEN
UNITE D'ADMINISTRATION MANUELLE OU AUTOMATIQUE DE PREPARATIONS INJECTABLES

(30) Priority: 18.12.2003 AR P030104719
(43) Date of publication of application: 06.09.2006
(73) Proprietor: Elastomeric Systems, S.L., 28043 Madrid (ES)
(72) Inventor: ROLLA, Jose Santiago, Lujan 670 Provincia de Buenos Aires (AR)
(74) Representative: Arpe Fernandez, Manuel de
(86) International application number: PCT/ES2004/000409
(87) International publication number: WO 2005/061029

(56) References cited:
- WO-A1-99/07421
- GB-A- 786 931
- US-A- 4 246 898
- US-A- 4 642 102
- US-B1- 6 562 007

## Description

### FIELD OF INVENTION AND DESCRIPTION OF PRIOR ART

The present invention consists in a unit to administer injectable medication manually or automatically, which can be used both manually and automatically within devices and machines.

Medication is injected by means of syringes in which generally the medication to be injected must be drawn form vials. This operation is done manually, which, besides being arduous and slow, frequently causes the contamination of the medication basically due to its contact with ambient air, and which often presents other technical deficiencies.

Generally, all the systems and methods of administering medication have not been adapted to the evolution experienced by the medical and welfare technologies. At present, more than 80% of the world injectable medication is given in a personalized and scheduled manner, in the case of inpatients, and it is administered intravenously for periods, which vary according to the status of the patient and the criterion of the doctor. This routine also consists in drawing the medication manually from the vials or bottles into the injection syringes, and from there, into the containers of sterile solutions for intravenous use. These manual procedures used in conventional syringes with the medication to be administered are carried out in non-sterile environments and, besides being very slow and complex, they are technically unsafe and involve a high risk due to the danger of the possibility of contamination because the injectable medication is in contact with non-sterile ambient air, and other technical faults such as possible mistakes in the dosage or incorrect identification of the medication or of the patients to whom it should be given. As a result of this deficient technology, frequent complications arise which worsen the patient status, which is generally followed by lawsuits for malpractice against medical institutions and doctors. Besides these serious inconveniences, these methods involve complicated, slow and inefficient administrative controls of the used and stored medication and of their invoicing processes. As can be clearly inferred, with this technology for drawing medication under conditions with no guaranteed asepsis, no medical institution of the world is ready to comply with ISO quality control standards as regards controls and methodology related to the administration of injectable medication.

From the point of view of ecology, with current systems and due to the dispersion of the elements used, consideration should be given to the environmental contamination caused by syringes, their needles and protective caps, glass vials and used flexible plastic containers, and to another serious risk of contamination implied by the possibility of reusing disposable syringes. Furthermore, the risk to which health care workers and all the staff handling medical refuse are exposed is very serious, due to the possibility of suffering accidental needle stick injuries occurring with needles contaminated with AIDS or other serious infectious diseases which inpatients might suffer and which are frequent in medical institutions.

There are various well known manual, automatic or semi-automatic systems of injecting medication in a single or multiple way, and syringes aimed at optimizing the application and transfer of injectable medication, such as those disclosed in the following patents: U. S. 3, 670, 923, U.S. 4,111,335, U.S. 4,333,356 U.S.N' 4,467,844, U.S. 4,509,861, U.S.N' 4,513,796; 4,527,245; U.S. 4,625,494, U.S. 5,431,201, UK GB 2,061,747; PCT/SE97/01094, EP 0 335 378 A2; EP 0 882 441 A2, EP 0 898 951 A2, U.S. 6 003 566; U.S. 6 039 093, and PCT/SE 00/01445. For more than fifty years, odontologists all over the world have been using a system which contains injectable anesthetic in a tubular element within a space determined between two plungers, but a special syringe with a piston and a perforation system of the lower plunger is needed to inject its content.

The patents and systems mentioned and various others develop multiple syringes and systems to mix and administer automatically or semi-automatically injectable or oral solutions, but since they fail to provide satisfactory solutions to current needs, none of them has been implemented and made known massively.

GB 786931 A to be considered as the prior art closest to the subject matter of the application, disclose a hypodermic syringe gauge, specifically with a gauging or controlling device, which can be readily attached to a hypodermic syringe and can be readily adjusted or set to determine the amount of liquid, which can be drawn into syringe so that it can later be ejected from the syringe. The hypodermic syringe is used to administer injectable medication manually or automatically which, being suitable for both manual use and for use in machines or devices, may contain a medicinal solution to be administered in one or more doses, and which is disposable once said solution has been completely used, and having: (a) a tubular body which, between a command end and an injection nozzle, forms a container receptacle, which contains the medicinal solution; (b) On its opposing ends, the receptacle is delimited by a plunger impelling the solution and a bottom formed by internal bottom walls of the tubular body; (c) said impelling plunge is able to be connected to manual or automatic action rods.

Here it is not foreseen that the plunger can be detached from the action rod. This means that the hypodermic syringe cannot be used as a permanent container for the injectable medicinal solution with sufficiently reduced dimensions to be stored until it is used and neither as a safe container for the transport of the injectable solution since when the syringe is filled with the medicinal solution, the plunger rod protrudes from the tubular body. The bottom part located beside the injection nozzle is formed by the inner walls of the tubular body and, therefore, is fixed and cannot be displaced to face the drilling means which are neither foreseen; consequently, as soon as the plunger is pushed by the rod, the injectable medical solution is impelled outside through the hypodermic needle and, therefore, it can neither be used as a permanent and safe container for the medicinal solution.

### SUMMARY OF THE INVENTION

The above-mentioned problems are achieved providing a unit to administer injectable medication manually according to the characteristics in claim 1.

The corresponding unit to administer injectable medication manually or automatically which, being suitable for both manual use and for use in machines or devices, may contain a medicinal solution to be administered in one or more doses, and which is disposable once said solution has been completely used, having:
b) A tubular body (2) which, between a command end (2a) and an injection nozzle (3), forms a container receptacle (6), which contains the medicinal solution;
characterized in that:
b) On its opposing ends, the container receptacle (6), is delimited by a plunger (4) impelling the solution and a drillable bottom (5);
c) said impelling plunger (4) is able to be connected to manual or automatic action rods (10);
d) inside the injection nozzle (3) there is a perforating means (7) which faces said drillable bottom (5); and
e) said perforating means (7) communicates with the outside of the receptacle through the injection nozzle (3).

The present invention efficiently solves all these inconveniences, thus beginning the first stage of a new technology related to the safe administration of injectable medication subcutaneously, without being drawn in and out, thus guaranteeing absolute sterility. The versatility of the system allows to begin the first stage of a new intravenous medication application system providing the possibility of automatically bottling, in an absolutely sterile system, the injectable medication scheduled in a personalized manner, keeping its sterility and avoiding the possibility of any kind of mistake, by means of electronic controls using bar code reading sensors and the like, which control all the steps in the transfer on injectable medication into the containers of intravenous solutions. To achieve this, the present invention provides manual or automatic injection of medication by means of new elements which constitute "Injecting Units" (IUs), in which the contained liquid medication and the thin tubular element from which the injectable medication flows are bottled jointly under sterility conditions during the manufacturing process, and which due to their special design and internal devices are excellent for these and other purposes.

The IUs consist in practical and efficient devices, which practically, safely and efficiently replace the joint function of vials and syringes currently used, allowing the direct fast injection of medication manually or through automatic systems, inside the containers of intravenous solutions. Each one of them may contain one or multiple doses of injectable medication.

In the case of emergency injectable medication of non-scheduled application in which the injection must be administered through elastomer diaphragms of the tubular elements of intravenous injection, which regulate the flow of medication, the present invention allows to do this using the element which enables the manipulation of the (IU), through a manufacturing variant wherein the lower end of the tubular element along which the injectable medication is transferred ends in a sharp pointed tip which, by means of enough pressure exerted manually using a piston, perforated the elastomer body to allow the flow of the injectable medication.

As it can be appreciated, this system provides speed and absolute safety in the administration of injectable medication, minimizing the possibility of human mistakes involved in the manual transfer, avoiding contamination of the medication by being in contact with ambient air and due to technical failures, and the mistakes in the medication administration, since on the surface of the (IU) the name is printed using letters that can be read with the naked eye or bar codes or other automatic reading systems of identification, as well as inscriptions which, by being read, allow to know the place and position of the (IU) so as to be used in the automatic system.

As regards environment and environmental care, this invention makes it impossible for health care workers and the staff handling medical, refuse to suffer accidental injuries or other types of damages caused by contaminated needles. Moreover, it prevents the injecting unit from being reused, since it immediately becomes useless after being used, and it allows for the absolute joint recycling of glass and plastic materials, elastomer and any other material used in the manufacture of the injecting units.

Another important advantage of the present invention is that by joining in a single element the ones that are currently used separately, it simplifies and makes the industrial bottling processes of injectable medication cheaper, by reducing the amounts of materials used in the manufacturing process and, thus, storing and transport spaces and costs.

This invention reduces the chances of human or other kinds of mistakes in the injectable medication application process, in every moment of the manual injection process or the programmed and personalized automatic transfer and bottling process, further preventing the injectable medication from being in direct contact with non-sterile ambient air, which constitutes an important contaminating factor.

The Injecting Units may also contain amino acids, liquid foods and other sterilized compounds to be transferred and mixed into sterile containers to be orally, injectable or otherwise administered. They are ideal to prepare doses and mix solutions used for renal dialysis. In other types of fluid or viscous substance difficult to handle and that should be mixed.

The (IU) allow to inject non-scheduled emergency medication through the elastomer membranes of tubular systems of intravenous application. They are also ideal for the application of vaccines or other medication dispensed subcutaneously. Furthermore, the contained injectable medication can be transferred under absolute sterility conditions into the usual disposable syringes, to be injected conventionally, by intramuscular or direct intravenous injection.

However, in the present invention as opposed form as related in prior art, the injecting unit autonomously and permanently contains the plunger perforation system and the medication injection or transfer system.

### BRIEF DESCRIPTION OF THE DRAWINGS

For the sake of clarity and to understand the object of the invention, it is illustrated in different figures in which it has been represented in one of the preferred embodiments, which is purely exemplary and therefore non-limiting:

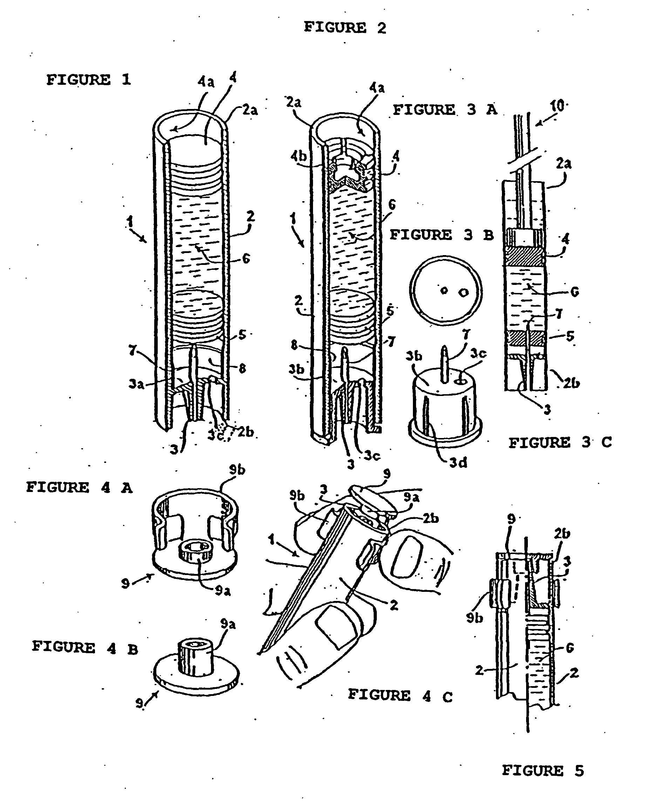

Figure 1 is a longitudinal view of the injecting unit in an embodiment suitable for automatic or manual use.
Figure 2 is a longitudinal view of another embodiment suitable for automatic or manual use.
Figure 3A is a longitudinal view showing the forward movement of an automatic action rod.
Figure 3B is an elevation top view of the fixable part in which the perforating means and the injection nozzle are situated.
Figure 3C is an elevation side view of the part in Figure 3B.
Figure 4A is a perspective view of a removable cap with elastic clamp.
Figure 4B is a perspective view of a removable cap without elastic clamp.
Figure 4C is a perspective view showing the application of the cap to the injection nozzle.
Figure 5 is a longitudinal view of the tubular body with the applied cap.

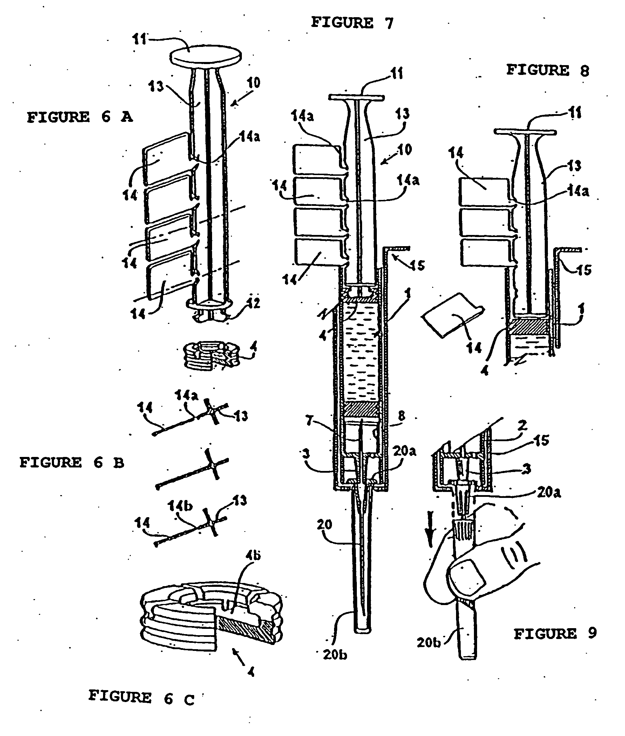

Figure 6A is a perspective view of a rod provided with stroke stops for dosing and an impelling plunger provided with coupling means to the rod.
Figure 6B is a cross-sectional view of the elongated body of the rod according to section lines indicated in Figure 6A.
Figure 6C is a cross-sectional view of the impelling plunger showing the coupling means to the rod.
Figure 7 is a longitudinal view of a manual application set including the injecting unit, the additional support and an injection needle.
Figure 8 is another longitudinal view showing how the stroke stops are used.
Figure 9 is another longitudinal view showing the injection needle arrangement.

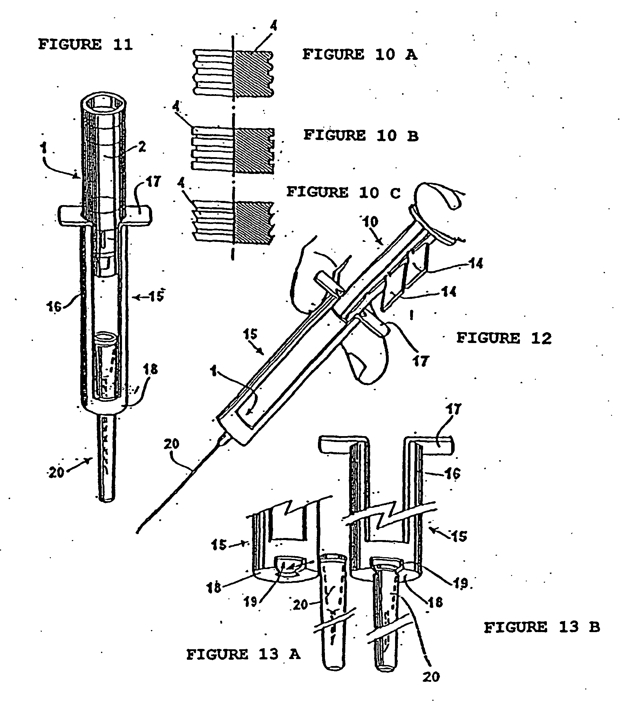

Figure 10A is a longitudinal view of a plunger (in the case of a drillable bottom, it would be similar) whit sealing means having a rounded projecting section.
Figure 10B is another longitudinal view wherein the sealing means have a rectangular projecting section.
Figure 10C is another longitudinal view wherein the sealing means have an angular projecting section.
Figure 11 is a perspective view illustrating the arrangement of the injecting unit and its needle in an additional support.
Figure 12 is another perspective view as the one in Figure 11, but in which the rod has been added, thus forming the set ready for manual use.
Figure 13A is a perspective view of the fitting recess of the additional support.
Figure 13B is another perspective view in which the needle head is fitted in the recess.

In the different figures, similar reference numbers and/or letters designate similar or corresponding parts.

### DETAILED DESCRIPTION OF THE EMBODIMENTS

The present invention consists in a unit to administer injectable medication manually or automatically which, in general terms, comprises a tubular body (2) with a receptacle (6) containing a solution, said receptacle (6) is delimited by an impelling plunger (4) and a drillable bottom (5); the drillable bottom (5) faces a perforating means (7) communicated with an injection nozzle (3); the impelling plunger (4) can be connected to manual or automatic action rods (10).

More particularly, the present unit (1) to administer injectable medication can be used both manually or in machines or devices used for automatically dosing, mixing or dispensing injectable solutions. Each unit (1) contains a medical solution, which can be applied in one or more doses, and it is disposable once said solution has been completely applied.

The present injecting unit (1) comprises a tubular body (2) which on one end finishes in a command end (2a) and on the opposite end finishes in an injection end (2b).

Inside the tubular body (2) there is an impelling plunger (4) and a drillable bottom (5), between which a receptacle (6) containing the injectable solution is defined.

Said drillable bottom (5) faces a perforating means (7) located at an end wall (3a), from which an injection nozzle (7) is formed. Inside there is a passage communicating the perforating means (7) with said injection nozzle (3). Furthermore, there is an air outlet (3c), which allows the drillable bottom (5) to move towards the perforating means (7).

The perforating means (7) may consist of an internal needle or a tubular element thicker than the sharp end.

The addition of retentive means (8) of the drillable bottom (5) has been provided. These retentive means (8) may consist in a sectional narrowing of the tubular body (2) so that the drillable bottom (5) may move forwards but not backwards.

The injection nozzle (3) is sunk within the limits of the tubular body (2) walls, without projecting with respect to the injection end (2b).

The end stop wall (3a, 3b), in which the perforating means (7) and the injection nozzle (3) are located, may be formed (3a) by the tubular body (2) proper, or it may consist in a part fixed (3b) by fixing means (3d) to the injection end (2b).

The injection nozzle (3) is adapted to be connected to needles (20), cannulas, tubes or any other equivalent element.

A removable cap (9) has also been provided. This cap (9) has a funnel (9a) in which the nozzle (3) is fitted, and a resilient clamp (9b), which allows to fix it to the tubular body (2).

Furthermore, the impelling plunger (4) is slightly away from the command end (2a) so that a guiding cavity (4a) is formed through which the command rod (10) enters.

The present injecting unit (1) is suitable for use both with automatic action rods (10), which are part of machines or devices and manual action rods (10). In the case of manual use, the impelling plunger (4) maybe coupling means (4b) for example, notched-grooved, which allow the connection with corresponding coupling means (12) provided by the rod (10).

It has also been provided that the elongated body (13) of the rod (10) has disposable stroke stops (14) sequentially arranged along the elongated body (13), so that each used stroke stop (14) enables the forward movement of the impelling plunger (4) and the administration of a corresponding dose. Preferably, the stroke stops (14) will have weakening lines (14b) in the cutting areas (14a), which will make their removal easier.

The use of an additional support (15) has also been provided to accommodate the injecting unit (1). This additional support (15), is formed by branching walls (16) forming longitudinal openings through which the injecting unit (1) is exposed. The branching walls (16) end in two side lugs (17) and, in the opposite end, they converge into an injection end (18). Said injection end (18) has a recess (19) suitable for the head (20a) of a needle (20) to fit in.

Both the impelling plunger (4) and the drillable bottom (5) have sealing means, which act against the tubular walls (2) of the injecting unit (1). These sealing means consist of an annular grooving, the ledges of which may be of rounded, rectangular or angular section, the sloping side of which is arranged so that it helps with the forward movement inside the tubular body (2).

It is apparent that when the present invention is put into practice, modifications may be made regarding certain construction and shape details, without departing from the basic principles, which are clearly encompassed in the following claims.

### LIST OF MAIN REFERENCES IN THE DRAWINGS:

(1) Injecting unit.
(2) Tubular body.
   (2a) Command end of the tubular body (2).
   (2b) Injection end of the tubular body (2).
(3) Injection nozzle.
   (3a) Formed end wall.
   (3b) Fixed end wall.
   (3c) Air outlet.
   (3d) Fixing means of the fixed end wall (3b).
(4) Impelling plunger.
   (4a) Guiding cavity.
   (4b) Coupling means to the rod (10).
(5) Drillable bottom.
(6) Receptacle for medicinal solution.
(7) Perforating means.
(8) Retentive means of the drillable bottom (5).
(9) Removable cap.
   (9a) Mouth for the injection nozzle (3).
   (9b) Elastic clamp.
(10) Rod.
(11) Rod lug (10).
(12) Coupling means to the impelling plunger (4).
(13) Elongated body of the rod (10).
(14) Disposable stroke stops (dosing means).
   (14a) Cutting area of the stroke stops (14).
   (14b) Weakening lines in the cutting areas (14a).
(15) Additional support.
(16) Branching walls of the additional support (15).
(17) Side lugs of the additional support (15).
(18) Injection end of the additional support (15).
(19) Needle fitting recess (20).
(20) Injection needle.
   (20a) Needle head (20).
   (20b) Needle protective cap (20).

## Claims

1. Unit to administer injectable medication manually or automatically which, being suitable for both manual use and for use in machines or devices, may contain a medicinal solution to be administered in one or more doses, and which is disposable once said solution has been completely used, and having:
a) A tubular body **(2)** which, between a command end **(2a)** and an injection nozzle **(3),** forms a container receptacle **(6),** which contains the medicinal solution;
**characterized in that**,
b) On its opposing ends, the receptacle is delimited by a plunger **(4)** impelling the solution when is actuated through a separated action rod **(10**) and a drillable bottom **(5)** shaped as a piston, located movable inside the tubular body **(2)**,
c) Said impelling plunger **(4)** is able to be connected to manual or automatic action rods **(10)** ;
d) Inside the tubular body, between the movable drillable bottom and the injection nozzle, there is a perforating means **(7)** which faces said drillable bottom, which perforating means serve to perforate said drillable bottom when the drillable bottom is moved impelled by the medicinal solution and said plunger is actuated, and in this way said medicinal solution contained between the plunger and the drillable bottom may flow outward; and
e) Said perforating means **(7)** form a duct to communicate the receptacle with the outside through the injection nozzle, so that the medicinal solution can flow.

2. Unit to administer injectable medication manually or automatically according to claim 1 **characterized in that** the plunger **(4)** has coupling means **(4b)** to the end of a command rod.

3. Unit to administer injectable medication manually or automatically according to claim 2 **characterized in that** between the plunger and the end of the command rod there are notched-grooved coupling means.

4. Unit to administer injectable medication manually or automatically according to claim 1 **characterized in that** between the command end of the receptacle and the plunger there is a guiding cavity **(4a)** defined for a command rod.

5. Unit to administer injectable medication manually or automatically according to claim 1 **characterized in that** the perforating means **(7)** is a needle.

6. Unit to administer injectable medication manually or automatically according to claim 1, which around the perforating means **(7)**, the receptacle has retentive means **(8)** of the drillable bottom **(5)** so that it allows the movement towards the nozzle **(3)** and prevents the movement backwards.

7. Unit to administer injectable medication manually or automatically according to claim 1 **characterized in that** adjacent to the nozzle **(3)** there is an air outlet **(3c)** of the receptacle which communicates the outside to the variable cavity formed between the drillable bottom **(5)** and said nozzle.

8. Unit to administer injectable medication manually or automatically according to claim 1 **characterized in that** the nozzle **(3)** is sunk within the limits of the tubular body walls, without projecting with respect to the injection end **(2b)** of said tubular body **(2).**

9. Unit to administer injectable medication manually or automatically according to claim 1 **characterized in that** the perforating means **(7)** and the injection nozzle **(3)** are on a stop end wall **(3b)** which is the advance limit of the drillable wall inside the receptacle.

10. Unit to administer injectable medication manually or automatically according to claim 9 **characterized in that** the stop end wall **(3b)** is formed by the tubular body **(2)**.

11. Unit to administer injectable medication manually or automatically according to claim 9 **characterized in that** the stop end wall **(3b)** is fixed to the injection end of the tubular body.

12. Unit to administer injectable medication manually or automatically according to claim 1 **characterized in that** the nozzle **(3)** is prepared to be connected to needles, cannulas, tubes and the like.

13. Unit to administer injectable medication manually or automatically according to claim 1 **characterized in that** the nozzle **(3)** has a removable cap **(9).**

14. Unit to administer injectable medication manually or automatically according to claim 13 **characterized in that** the cap **(9)** fits in the nozzle **(3)** and has a resilient clamp **(9b)** as a fixing means to the end adjacent to the tubular body **(2)**.

15. Unit to administer injectable medication manually or automatically according to claim 1 **characterized in that** the command rod **(10)** has, at least, one non-reusable stroke stop **(14)** as a dosing means.

16. Unit to administer injectable medication manually or automatically according to claim 1
**characterized in that** the command rod **(10)** has plural non-reusable stroke stops **(14),** sequentially arranged along said rod.

17. Unit to administer injectable medication manually or automatically according to claim 15 **characterized in that** the stroke stops have respective weakening lines **(14a)** that determine the cutting area and removal with respect to the body **(13)** of the command rod **(10)**.

18. Unit to administer injectable medication manually or automatically according to claim 1 **characterized in that** having an additional support **(15)** which accommodates the tubular body **(10)** and which, on one end, forms a handgrip means **(17)** while, on the opposite end, forms an injection end **(18)** against which the nozzle **(3)** is arranged.

19. Unit to administer injectable medication manually or automatically according to claim 18 **characterized in that** the injection end **(18),** against which the nozzle **(3)** is arranged, forms a recess **(19)** prepared for the head **(20a)** of a needle **(20)** to fit in.

20. Unit to administer injectable medication manually or automatically according to claim 18
**characterized in that** the additional support **(15)** forms a housing open lengthwise for the tubular body **(2)**; the longitudinal openings are delimited by branching walls **(16)** which start at the injection end **(18)** and finish in two lugs **(17)**.

21. Unit to administer injectable medication manually or automatically according to claim 1
**characterized in that** the impelling plunger **(4)** has sealing means consisting of an annular grooving.

22. Unit to administer injectable medication manually or automatically according to claim 1
**characterized in that** the drillable bottom **(5)** has sealing means consisting of an annular grooving.

23. Unit to administer injectable medication manually or automatically according to claims 21 and 22 **characterized in that** the annular grooving forms ledges of rounded section.

24. Unit to administer injectable medication manually or automatically according to claims 21 and 22 **characterized in that** the annular grooving forms ledges of rectangular section.

25. Unit to administer injectable medication manually or automatically according to claims 21 and 22 **characterized in that** the annular grooving forms ledges of angular section, the sloping side of which is arranged so that it helps with the forward movement inside the tubular body **(2)**.

## Patentansprüche

1. Einheit zur manuellen oder automatischen Verabreichung von injizierbaren Arzneimitteln, welche geeignet ist für den manuellen Gebrauch, wie auch zur Anwendung in automatischen Maschinen oder Vorrichtungen, beinhaltet eine in einer oder mehr Dosis zu verabreichenden Lösung zum medizinischen Gebrauch, wobei die Einheit nach Verbrauch der beinhalteten Lösung zu entsorgen ist und welche einschließt:
a) einen rohrförmigen Körper (2), welcher zwischen einem Betätigungsende (2 a) und einer Injektionsdüse (3) eine Kammer (6) zur Aufnahme von der Injektionslösung zum medizinischen Gebrauch bildet, **dadurch gekennzeichnet,**
b) **dass** an den gegenüberliegenden Enden die Kammer durch einen Druckkolben (4) zur Injizierung der Lösung, wenn er mittels eines Schafts mit separater Wirkung (10) betätigt wird, und eines durchstechbaren Bodens (5) in der Art eines Stempels begrenzt wird, der sich beweglich innerhalb des rohrförmigen Körpers (2) befindet;
c) **dass** jener Druckkolben (4) mit Schäften manueller oder automatischer Betätigung (10) verbunden werden kann;
d) **dass** sich innerhalb des rohrförmigen Körpers zwischen dem beweglichen Durchstechboden und der Injektionsdüse, ein Durchstechelement (7) gegenüber jenes Durchstechbodens befindet, deren Durchstechelement jenen Durchstechboden durchsticht, wenn dieser sich von der Lösung medizinischen Gebrauchs angetrieben verschiebt, wobei gleichzeitig deren Schaft betätigt wird und so jene sich zwischen dem Kolben und dem durchstechbaren Boden befindliche Lösung medizinischen Gebrauchs ausgestossen werden kann; und
e) **dass** jenes Durchstichelement (7) eine Leitung zur Verbindung der Kammer mit dem Außenbereich über die Injektionsdüse bildet, damit der Fluss der Lösung ermöglicht wird.

2. Einheit zur manuellen oder mechanischen Verabreichung von Injektionspräparatengemäß Anspruch 1, **dadurch gekennzeichnet, dass** der Kolben (4) Kupplungselemente (1b) zur Kupplung mit dem Ende des Betätigungsschafts aufweist.

3. Einheit zur manuellen oder mechanischen Verabreichung von Injektionspräparaten gemäß Anspruch 2, **dadurch gekennzeichnet, dass** sich zwischen dem Kolben (4) und dem Ende des Betätigungsschafts (10) Kupplungselemente in der Art einer Verzahnung befinden.

4. Einheit zur manuellen oder mechanischen Verabreichung von Injektionspräparaten gemäß Anspruch 1, **dadurch gekennzeichnet, dass** zwischen dem Betätigungsende der Kammer und der Kupplung ein Leitraum (4 a) für einen Betätigungsschaft gebildet wird.

5. Einheit zur manuellen oder mechanischen Verabreichung von Injektionspräparaten gemäß Anspruch 1, **dadurch gekennzeichnet, dass** das Durchstechelement (7) aus einer Nadel besteht.

6. Einheit zur manuellen oder mechanischen Verabreichung von Injektionspräparaten gemäß Anspruch 1, **dadurch gekennzeichnet, dass** rund um das Durchstechelement (7), die Kammer Halteelemente (8) des durchstechbaren Bodens (5) aufweist, so dass sein Fortbewegen bis zur Düse (3) ermöglicht und eine Rückkehr verhindert wird.

7. Einheit zur manuellen oder mechanischen Verabreichung von Injektionspräparaten gemäß Anspruch 7, **dadurch gekennzeichnet, dass** sich neben der Düse (3) ein Luftaustritt (3c) der Kammer befindet, welche die veränderbare zwischen dem durchstechbaren Boden (5) y jener Düse gebildeten Kammer mit der äußeren Umgebung verbindet.

8. Einheit zur manuellen oder mechanischen Verabreichung von Injektionspräparaten gemäß Anspruch 1 , **dadurch gekennzeichnet, dass** die Düse (3) innerhalb der Abgrenzungen des rohrförmigen Körpers (2) liegt, ohne bezüglich des Injektionsendes (2 b) jenes rohrförmigen Körpers (2) hervorzustehen.

9. Einheit zur manuellen oder mechanischen Verabreichung von Injektionspräparaten gemäß Anspruch 1, **dadurch gekennzeichnet, dass** das Durchstechelement (7) und die Injektionsdüse (3) sich an der äußeren Anschlagwandung (3b) befinden, welche die Grenze der Fortbewegung der durchstechbaren Wand innerhalb der Kammer darstellt.

10. Einheit zur manuellen oder mechanischen Verabreichung von Injektionspräparaten gemäß Anspruch 9, **dadurch gekennzeichnet, dass** die äußere Anschlagwandung (3b) von einem rohrförmigen Körper (2) gebildet wird.

11. Einheit zur manuellen oder mechanischen Verabreichung von Injektionspräparaten gemäß Anspruch 9, **dadurch gekennzeichnet, dass** die äußere Anschlagwandung (3b) am Injektionsende des rohrförmigen Körpers fixiert ist.

12. Einheit zur manuellen oder mechanischen Verabreichung von Injektionspräparaten gemäß Anspruch 1, **dadurch gekennzeichnet, dass** die Düse (3) für die Verbindung mit Nadeln, Kanülen, Röhren oder ähnliches vorbereitet ist.

13. Einheit zur manuellen oder mechanischen Verabreichung von Injektionspräparaten gemäß Anspruch 1, **dadurch gekennzeichnet, dass** die Düse über einen aufsetz- und abnehmbaren Deckel (9) verfügt.

14. Einheit zur manuellen oder mechanischen Verabreichung von Injektionspräparaten gemäß Anspruch 13, **dadurch gekennzeichnet, dass** der Deckel (9) auf die Düse (3) passt und mit einer elastischen Schelle (9b) als Mittel zur Fixierung an das nebenstehende Ende des rohrförmigen Körpers versehen ist.

15. Einheit zur manuellen oder mechanischen Verabreichung von Injektionspräparaten gemäß Anspruch 1, **dadurch gekennzeichnet, dass** der Betätigungsschaft (10) über mindestens einen entsorgbaren Verschiebungsanschlag (14) als Dosierungsmittel verfügt.

16. Einheit zur manuellen oder mechanischen Verabreichung von Injektionspräparaten gemäß Anspruch 1, **dadurch gekennzeichnet, dass** der Betätigungsschaft (10) über eine Vielzahl von entsorgbaren Verschiebungsanschlägen (14) verfügt, die sukzessive in Längsrichtung des Betätigungsschaft vorgesehen sind.

17. Einheit zur manuellen oder mechanischen Verabreichung von Injektionspräparaten gemäß Anspruch 15, **dadurch gekennzeichnet, dass** die Verschiebungsanschläge gestanzte Linien (14a) zur Bestimmung der Riss- und Abnahme bezüglich des Körpers (13) des Betätigungsschafts (10) aufweisen.

18. Einheit zur manuellen oder mechanischen Verabreichung von Injektionspräparaten gemäß Anspruch 1, **dadurch gekennzeichnet, dass** eine Zusatzhalter (15) zur Aufnahme des rohrförmigen Körpers (10) vorgesehen ist, und dass diese einerseits ein seitliches Halteelement (17) bildet während am gegenüberliegenden Ende ein Injektionsende (18) gebildet wird, gegen welches die Düse (3) zum Anliegen kommt.

19. Einheit zur manuellen oder mechanischen Verabreichung von Injektionspräparaten gemäß Anspruch 18, **dadurch gekennzeichnet, dass** das Injektionsende (18), gegen welches die Düse (3) zum Anliegen kommt, eine zur Einfügung des Kopfes (20a) einer Injektionsnadel (20) vorbereiteten Ausnehmung (19) aufweist.

20. Einheit zur manuellen oder mechanischen Verabreichung von Injektionspräparaten gemäß Anspruch 18, **dadurch gekennzeichnet, dass** der Zusatzhalter (15) eine in Längsrichtung offene Aufnahme für den zylindrischen Körper (2) bildet, die längsverlaufenden Öffnungen durch Verzweigungswandungen (16) begrenzt sind, die ab dem Injektionsende (18) jeweils in Halteelementen (17) enden.

21. Einheit zur manuellen oder mechanischen Verabreichung von Injektionspräparaten gemäß Anspruch 1, **dadurch gekennzeichnet, dass** der Betätigungsschaft (4) über aus ringförmigen Rillungen bestehende Dichtungselemente verfügt.

22. Einheit zur manuellen oder mechanischen Verabreichung von Injektionspräparaten gemäß Anspruch 1, **dadurch gekennzeichnet, dass** der Betätigungsschaft (5) über aus ringförmigen Rillungen bestehende Dichtungselemente verfügt.

23. Einheit zur manuellen oder mechanischen Verabreichung von Injektionspräparaten gemäß Ansprüchen 21 und 22, **dadurch gekennzeichnet, dass** die ringförmigen Rillungen ein abgerundetes hervorstendes Profil bilden.

24. Einheit zur manuellen oder mechanischen Verabreichung von Injektionspräparaten gemäß Ansprüchen 21 und 22, **dadurch gekennzeichnet, dass** die ringförmigen Rillungen ein rechteckiges hervorstendes Profil bilden.

25. Einheit zur manuellen oder mechanischen Verabreichung von Injektionspräparaten gemäß Ansprüchen 21 und 22, **dadurch gekennzeichnet, dass** die ringförmigen Rillungen ein winkeliges hervorstendes Profil bilden, dessen geneigte Seite so vorgesehen ist, dass sie bei der fortschreitenden Verschiebung innerhalb des rohrförmigen Körpers (2) mitwirkt.

## Revendications

1. Unité pour la provision de médicaments injectables de forme manuelle ou automatique, celle qui étant aptes pour l'utilisation manuelle ainsi que pour son emploi en machines automatiques ou dispositifs, contient une solution d'utilisation médicinale applicable a une ou plusieurs doses, celle qui est rejetée une fois poussée complètement la charge de ladite solution, elle est composée de
a) Un corps tubulaire (2) celui qui entre en extrême du commandement (2a) et l'embouchure d'injection (3) comporte un réceptacle qui contient (6) la solution pour l'utilisation médicinale
**Caractérisée en ce que**
b) Ce réceptacle qui contient (6) dans ses extrêmes opposés, est délimité par un piston (4) pousseur de la solution lorsqu'il est mis en mouvement par le piston d'action séparée (10) et un fond perforant (5) avec la forme d'un piston, placé de forme mobile dans le corps tubulaire (2) ;
c) Ce piston (4) pousseur est capable de se lier aux tiges d'action manuelle ou automatique (10) ;
d) Dans le corps tubulaire entre le fond perforant mobile et l'embouchure d'injection, il y a un moyen perforateur (7) qui fait face à ce fond pouvant être perforé, dont son moyen perforateur sert a perforer ledit fond perforant lorsque celui/ci est bougé poussé par la solution d'utilisation médicinale et ledit piston est actionné, et de cette manière la solution médicinal contenue entre le piston et le fond perforant peut glisser vers l'extérieur, et
e) Ce moyen perforateur (7) forme un conduit pour communique le réceptacle avec l'extérieur a travers de l'embouchure d'injection, pour que la solution puisse glisser.

2. Unité pour la provision d'injectables de forme manuelle ou automatique, selon la revendication 1, **caractérisée en ce que** le piston (4) a les moyens d'accouplement (4b}avec l'extrême du piston de commandement.

3. Unité pour la provision d'injectables de forme manuelle ou automatique, selon la revendication 2, **caractérisée en ce que** entre le piston (4) et l'extrême du piston de commandement (10), il y a des moyens d'accouplement (4b) du genre dent/rainure.

4. Unité pour la provision d'injectables de forme manuelle ou automatique, selon la revendication 1, **caractérisée en ce que** entre l'extrême de commandement du réceptacle et le piston reste définie une cavité de guide (4a) pour un piston de commandement.

5. Unité pour la provision d'injectables de forme manuelle ou automatique, selon la revendication 1, **caractérisée en ce que** le moyen perforateur (7) consiste en une aiguille.

6. Unité pour la provision d'injectables de forme manuelle ou automatique, selon la revendication 1, **caractérisée en ce qu'**autour du moyen perforateur (7) le réceptacle dispose de moyens de rétention (8} du fond perforant (5) de manière à habiliter son avancement vers l'embouchure (3) et empêcher son recul.

7. Unité pour la provision d'injectables de forme manuelle ou automatique, selon la revendication 1, **caractérisée en ce que** de manière adjacente par rapport à l'embouchure (3) il y a une sortie d'air (3c) du réceptacle qui communique avec l'extérieur à la cavité variable formée entre le fond perforant (5) et cette embouchure.

8. Unité pour la provision d'injectables de forme manuelle ou automatique, selon la revendication 1, **caractérisée en ce que** l'embouchure (3) est foncée dans les limites des murs du corps tubulaire, sans surpasser par rapport à l'extrême d'injection (2b) dudit corps tubulaire (2).

9. Unité pour la provision d'injectables de forme manuelle ou automatique, selon la revendication 1, **caractérisée en ce que** le moyen perforateur (7) et l'embouchure d'injection (3) sont sur le mur extrême de l'arrêt (3b) qui constitue la limite d'avancement du mur perforant dans le réceptacle.

10. Unité pour la provision d'injectables de forme manuelle ou automatique, d'après la revendication 9, **caractérisée en ce que** le mur extrême d'arrêt (3b) est composé par le corps tubulaire (2).

11. Unité pour la provision d'injectables de forme manuelle ou automatique, selon la revendication 9, **caractérisée en ce que** le mur extrême d'arrêt (3b) est fixé à l'extrême d'injection du corps tubulaire.

12. Unité pour la provision d'injectables de forme manuelle ou automatique, selon la revendication 1, **caractérisée en ce que** l'embouchure (3) est préparée pour la connexion avec les aiguilles, canules, tubes ou équivalents.

13. Unité pour la provision d'injectables de forme manuelle ou automatique, selon la revendication 1, **caractérisée en ce que** l'embouchure (3) dispose d'un couvercle démontable (9).

14. Unité pour la provision d'injectables de forme manuelle ou automatique, selon la revendication 13, **caractérisée en ce que** le couvercle (9) entre dans l'embouchure (3) et a un anneaux élastique flexible (9b) comme moyen de fixation à l'extrême adjacent du corps tubulaire.

15. Unité pour la provision d'injectables de forme manuelle ou automatique, selon la revendication 1, **caractérisée en ce que** la tige de commandement (10) dispose de, au moins un arrêt rejetable (14) en qualité de moyen doseur.

16. Unité pour la provision d'injectables de forme manuelle ou automatique, selon la revendication 1, **caractérisée en ce que** la tige de commandement (10) dispose d'une pluralité d' arrêts rejetables (14), de manière séquentielle disposés tout au long de ladite tige.

17. Unité pour la provision d'injectables de forme manuelle ou automatique, d'après la revendication 15, **caractérisée par**ce que les arrêts (14} ont les respectives lignes de faiblesse (14a) qui déterminent le lieu de la coupure et la séparation du corps (13) de la tige de commandement (10).

18. Unité pour la provision d'injectables de forme manuelle ou automatique, selon la revendication 1, **caractérisée en ce qu'**elle dispose d'un support accessoire (15) qui offre logement au corps tubulaire (10) et que, d'une part forme un extrême de poignée (17) en tant que, d'autre part forme une extrémité d'injection (18) contre laquelle reste disposée l'embouchure (3).

19. Unité pour la provision d'injectables de forme manuelle ou automatique, selon la revendication 18, **caractérisée en ce que** l'extrémité d'injection (18) contre laquelle reste disposée l'embouchure (3) formant un décolletage (19) préparée pour la mise de la tête (20a) d'une aiguille (20).

20. Unité pour la provision d'injectables de forme manuelle ou automatique, selon la revendication 18 **caractérisée en ce que** le support accessoire (15) forme un logement longitudinale ouvert pour le corps tubulaire (2) les ouvertures longitudinales résultant délimitées par des murs en guise de branchement (16), lesquelles sont nées à l'extrémité d'injection (18} finissant en tous les deux poignées (17).

21. Unité pour la provision d'injectables de forme manuelle ou automatique, selon la revendication 1, **caractérisée en ce que** le piston pousseur (4) dispose de moyens de scellement constitués par des rainures annulaires.

22. Unité pour la provision d'injectables de forme manuelle ou automatique, selon la revendication 1, **caractérisée en ce que** le piston pousseur (5) dispose de moyens de scellement constitués par des rainures annulaires.

23. Unité pour la provision d'injectables de forme manuelle ou automatique, selon les revendications 21 et 22, **caractérisée en ce que** les rainures annulaires forme des excès de profil arrondi.

24. Unité pour la provision d'injectables de forme manuelle ou automatique, selon les revendications 21 et 22, **caractérisée en ce que** les rainures annulaires forment des saillants de profil rectangulaire.

25. Unité pour la provision d'injectables de forme manuelle ou automatique, selon les revendications 21 et 22, **caractérisée par**ce que les rainures annulaires forment des saillants de profil anguleux dont le côté incliné reste disposé de telle manière qu'il aide le sens de l'avancement dans le corps tubulaire (2).
